# EUROPEAN PATENT SPECIFICATION

(11) **EP 2 692 307 B1**
(45) Date of publication and mention of the grant of the patent: **28.01.2015**
(21) Application number: 13178978.6
(22) Date of filing: 01.08.2013
(51) Int. Cl.: A61B 19/00

(54) **Device for supporting and for adjusting the position of a patient's head during surgeries**
Vorrichtung zur Unterstützung und zum Anpassen einer Position eines Patientenkopfes während einer chirurgischen Operation
Dispositif pour soutien et pour positionner la tête d'un patient pendant une opération chirurgicale

(30) Priority: 03.08.2012 IT MI20121378
(43) Date of publication of application: 05.02.2014
(73) Proprietor: Consiglio Nazionale Delle Ricerche, 00185 Roma (IT)
(72) Inventor: Malosio, Matteo, I-00185 Roma (IT); Negri, Simone Pio, I-00185 Roma (IT); Pedrocchi, Nicola, I-00185 Roma (IT); Molinari Tosatti, Lorenzo, I-00185 Roma (IT); Vicentini, Federico, I-00185 Roma (IT)
(74) Representative: Leihkauf, Steffen Falk

(56) References cited:
- DE-U1- 9 116 002
- US-A- 5 160 337
- US-A- 5 276 927
- US-A- 5 879 281
- US-A1- 2005 160 532
- US-A1- 2008 072 381
- US-B1- 6 594 839

## Description

The present invention relates generally to devices for supporting objects with the possibility of adjustment and control of the position of the object itself and, in particular, the present invention relates to a device for supporting and adjusting the position of a patient's head during surgeries or diagnostics.

Known devices for supporting the head during brain surgery, for example the so-called "*Mayfield Clamp*", comprise a clamp with two opposite jaws, one of which supports a first supporting portion and the other supports a second supporting portion and a third supporting portion spaced apart in such a way as to engage the patient's head in three different points and lock the position and orientation of the head relative to the clamp itself. In order to allow an adaptation of the clamp to the patient's cranial shape, both the relative position between the two jaws and the relative position between the individual support portions and the jaws to which they are connected are manually adjustable and lockable by means of adjustment screws. In addition to the locking of the patient's head to the clamp, it is known to constrain the "Mayfield Clamp" in relation to the operating table or to a bed on which the patient is lying, by means of a fastening which allows a manual adjustment of translational and rotational position of the clamp relative to the operating table or bed. In this way it is possible to hold the patient's head in a position chosen by the surgeon to facilitate the surgery or diagnostics to the brain. US-B1-6594839 discloses a head supporting device having the features of the preamble of claim 1.

While the prior art devices for supporting the head ensure high stiffness of the support, they do not allow an easy and precise adjustment of the position. In fact, because of the manual adjustment mechanisms that determine composite translational and rotary adjustment movements, it is almost never possible to obtain the desired positioning by unlocking and operating a single adjustment screw, but the adjustment operation may require the simultaneous adjustment of multiple adjustment screws and require the presence of multiple people at the operating table (at least one person to hold the patient's head and at least another person to adjust the position of the "Mayfield Clamp"). It is also desirable to further shorten the positioning time.

Furthermore, the passive nature of the known devices for supporting the head excludes a quick, precise and easy adjustment of the head position in particular conditions that can occur during the surgery, e.g. a voluntary or involuntary movement of the patient or the need for the surgeon to reposition the patient's head during the surgery.

Therefore, the need to be able to make a fine adjustment of the positioning of the patient's head is felt, preferably even a rotation only or translation only adjustment without simultaneously changing the position and orientation relative to the remaining translation and rotation axes. This would allow a posture and mobility of the joints and muscles of the neck more consistent with the physiological condition, by obviating improper stress and pain or fatigue of the patient during the surgery.

The need to be able to adjust the patient's head position with high speed and precision, as well as with high supporting rigidity of the head both during and after the adjustment is also felt. This would allow to respond in real time to stress or sudden voluntary or involuntary movements of the patient (for example, during "awake surgery" (surgery with patient awake) that might otherwise cause severe pain or negatively affect the surgical procedure.

The need to be able to position the head of patients undergoing surgery with less medical personnel present at the operating table is also felt.

Finally, the dimensions of the head supporting device must be reduced such as to not excessively protrude from the sides of the operating table and allow free side and top access above the patient's head.

The object of the present invention therefore is to provide a device for supporting and adjusting the position of a patient's head during surgery, said device having such features as to overcome the drawbacks mentioned with reference to the prior art.

Within the scope of the main object, a particular object of the invention is to provide a supporting and adjustment device which allows a fine adjustment of the positioning of the patient's head, and also rotation only or translation only adjustment without simultaneously modifying the position and orientation relative to the remaining translation and rotation axes.

A further particular object of the invention is to be able to adjust the position of the patient's head in accordance with the scope of the natural mobility of the joint and the muscles of the neck.

A further object of the invention is to be able to adjust the position of the patient's head with high speed and precision, as well as with high support rigidity of the head both during and after the adjustment.

A further object of the invention is to provide a device for supporting and adjusting the position of a patient's head during surgery, having such features as to obviate the need for multiple people for the adjustments of the head positions.

At least some of the objects are achieved through a device for supporting and adjusting the position of a patient's head during surgery, the device comprising:
- a supporting structure for supporting the device on an application (for example a floor or a base of an operating table), said supporting structure defining a first axis (longitudinal horizontal), a second axis (transverse horizontal) and a third axis (vertical) mutually orthogonal;
- a mobile platform defining a platform centre and being constrained to the supporting structure by an adjusting mechanism suitable to position the platform in relation to the supporting structure in a neutral position and to adjust the position of the platform in relation to the supporting structure,
- a gripping device connected to the platform and having locking means suitable to lock the head to the gripping device,
wherein the adjusting mechanism comprises:
- a first arm having a first saddle slidably connected to the supporting structure along a first trajectory and positionable along the first trajectory by a first actuator, as well as a first rod having a first end rotatably connected to the first saddle and a second end rotatably connected to the platform,
- a second arm having a second saddle slidably connected to the supporting structure along a second trajectory and positionable along the second trajectory by a second actuator, as well as a second rod having a first end rotatably connected to the second saddle and a second end rotatably connected to the platform,
- a third arm having a third saddle slidably connected to the supporting structure along a third trajectory and positionable along the third trajectory by a third actuator, as well as a third rod having a first end rotatably connected to the third saddle and a second end rotatably connected to the platform,
- a fourth arm having a fourth saddle slidably connected to the supporting structure along a fourth trajectory and positionable along the fourth trajectory by a fourth actuator, as well as a fourth rod having a first end rotatably connected to the fourth saddle and a second end rotatably connected to the platform,
- a fifth arm having a fifth saddle slidably connected to the supporting structure along a fifth trajectory and positionable along the fifth trajectory by a fifth actuator, as well as a fifth rod having a first end rotatably connected to the fifth saddle and a second end rotatably connected to the platform,
- wherein the platform and the first, second, third, fourth, and fifth arms form together an articulated frame displaceable by the first, second, third, fourth, and fifth actuators so that the platform can translate in the direction of the first axis, the second axis, and the third axis and rotate around the second axis and the third axis.

In this way, a parallel kinematics adjustment mechanism with five degrees of freedom with five arms operable automatically and independently is obtained.

A parallel kinematics with five degrees of freedom thus configured creates the conditions for a support of the platform and an active movement thereof with a high intrinsic rigidity and, therefore, with high reliability of maintaining the centre position of the platform and, therefore, of the gripping device that holds the patient's head in relation to the supporting structure.

The first, second, third, fourth and fifth actuators are operable to adjust in a controlled manner the position of the platform (and therefore the patient's head constrained thereto) in relation to the supporting structure.

This allows a fine adjustment of the position of the head of patients undergoing surgery, and in particular rotation only or translation only adjustments without simultaneously modifying the position and orientation of the head relative to the remaining translation and rotation axes.

Thanks to regulation by means of said parallel kinematics operated by five actuators, such as linear electric motors, it is possible to adjust the head position with high speed and precision, as well as with high support rigidity of the head during adjustment movements.

Moreover, thanks to the fact that the actuators are located on the opposite side of the rods relative to the platform, the overall dimensions of the device in the region of the platform and of the gripping device are minimal and the actuators can be easily positioned in a part of the supporting structure intended to be beneath the operating table and away from the patient's head. This allows free access to the head during brain surgery. In addition, the device allows the positioning of the head with less medical personnel present at the operating table.

In accordance with one aspect of the invention, the gripping device is connected to the platform in a rotatable manner around a local axis and orientable around the local axis in relation to the platform by means of a sixth actuator. The local axis passes through a point that is eccentric relative to the platform centre, and when the platform stands in the neutral position, the local axis is parallel to the first axis and is spaced apart from the platform centre in the direction of the third axis.

This introduces the sixth controllable degree of freedom (in series with respect to the five arms described above) which allows a rotation of the head with a twist of the neck close to the neck itself.

To better understand the invention and appreciate its advantages, some exemplary non-limiting embodiments thereof shall now be described with reference to the accompanying figures, in which:

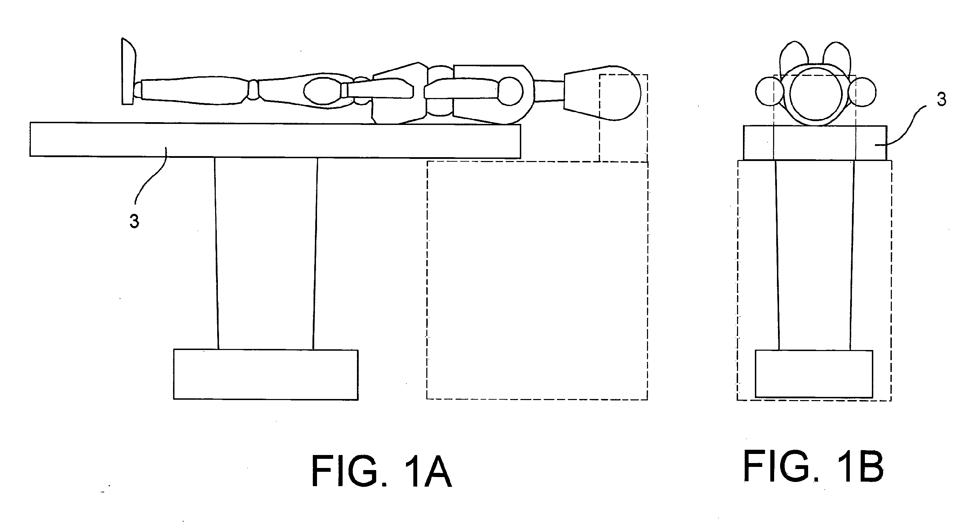

figures 1A and 1B show a patient lying on an operating table and with a dashed line they indicate the overall dimensions available recommended for any supporting structures of the head, to facilitate the presence of medical personnel and other equipment present during the surgery;
figure 2 shows an axonometric view of the device applied to an operating table and to a patient's head;
figures 3 and 5 show axonometric views of the device according to an embodiment;
figure 4 shows a top view of the device according to an embodiment;
figure 6A shows an axonometric side-rear view of the device in figure 5;
figure 6B shows the view in figure 12A with the addition of vectors which indicate translations and rotations of components of the arms of the adjusting mechanism;
figure 7A shows a top axonometric view of the device in figure 5;
figure 7B shows the view in figure 7A with the addition of vectors which indicate translations and rotations of components of the arms of the adjusting mechanism;
figure 8A shows an axonometric side-rear view of the device in figure 5;
figure 8B shows the view in figure 8A with the addition of vectors which indicate translations and rotations of components of the arms of the adjusting mechanism;
figure 9 shows a schematic topological representation with the indication of the kinematic connections of the adjusting mechanism of the head supporting device according to an embodiment;
figures 10, 11, 12, 13 and 14 show schematic topological representations of the adjusting mechanism of the head supporting device according to embodiments of the invention.

Referring to the figures, a device for supporting and adjusting the position of a patient's head during surgery is generally indicated with reference numeral 1. Device 1 comprises a supporting structure 2 resting device 1 on an application (for example a floor or a base of an operating table 3). The supporting structure 2 defines a first axis e_{x,W} (longitudinal horizontal), a second axis e_{y,W} (transverse horizontal) and a third axis e_{z,W} (vertical) mutually orthogonal.

Device 1 further comprises a mobile platform 4 defining a platform centre Oₘ and being constrained to the supporting structure 2 by an adjusting mechanism 5 suitable to position platform 4 in relation to the supporting structure 2 in a neutral position and to adjust the position of platform 4 in relation to the supporting structure 2.

Device 1 further comprises a gripping device 6 connected to platform 4 and having locking means 7 suitable to lock the head to the gripping device 6.

According to an aspect of the invention, the adjusting mechanism 5 comprises:
- a first arm 8 having a first saddle P₁ slidably connected to the supporting structure 2 along a first trajectory u_{1,1} and positionable along the first trajectory u_{1,1} by a first actuator 9, as well as a first rigid rod L_{1,1} having a first end rotatably connected to the first saddle P₁ and a second end rotatably connected to platform 4,
- a second arm 10 having a second saddle P₂ slidably connected to the supporting structure 2 along a second trajectory u_{2,1} and positionable along the second trajectory u_{2,1} by a second actuator 11, as well as a second rigid rod L_{2,1} having a first end rotatably connected to the second saddle P₂ and a second end rotatably connected to platform 4,
- a third arm 12 having a third saddle P₃ slidably connected to the supporting structure 2 along a third trajectory u_{3,1} and positionable along the third trajectory u_{3,1} by a third actuator 13, as well as a third rigid rod L_{3,1} having a first end rotatably connected to the third saddle P₃ and a second end rotatably connected to platform 4,
- a fourth arm 14 having a fourth saddle P₄ slidably connected to the supporting structure 2 along a fourth trajectory u_{4,1} and positionable along the fourth trajectory u_{4,1} by a fourth actuator 15, as well as a fourth rigid rod L₄ having a first end rotatably connected to the fourth saddle P₄ and a second end rotatably connected to platform 4,
- a fifth arm 16 having a fifth saddle P₅ slidably connected to the supporting structure 2 along a fifth trajectory u_{5,1} and positionable along the fifth trajectory u_{5,1} by a fifth actuator 17, as well as a fifth rigid rod L₅ having a first end rotatably connected to the fifth saddle P₅ and a second end rotatably connected to platform 4,
wherein platform 4 and the first 8, second 10, third 12, fourth 14 and fifth 16 arms form together an articulated frame displaceable by the first 9, second 11, third 13, fourth 15, and fifth 17 actuators so that the platform centre Oₘ can translate in the direction of the first axis e_{x,W}, the second axis e_{y,W}, and the third axis e_{z,W} and rotate around the second axis e_{y,W} and the third axis e_{z,W}. Although the rotations are described with reference to the global coordinates, the man skilled in the art will appreciate that if the platform is already at least slightly rotated about the second axis e_{Y},_{W} the rotation is not strictly around the third axis e_{Z,W} but around the vertical axis of platform 4 and its local coordinate system.

In this way, a parallel kinematics adjusting mechanism with five degrees of freedom with five arms automatically and independently operable is obtained. In the present description, the term trajectory means a guided movement path along which movement path the saddle can be locked and displaced forward and backward.

In accordance with an embodiment, the first 9, second 11, third 13, fourth 15 and fifth 17 actuators may for example include linear electric motors connected to the supporting structure 2 and acting respectively on the first ends of the first L_{1,1}, second L_{2,1}, third L₃, fourth L₄ and fifth L₅ and are operable in a controlled manner to adjust the position of platform 4 (and, therefore, the patient's head constrained to it) in relation to the supporting structure 2.

In accordance with an embodiment, the gripping device 6 is connected to platform 4 rotatably around a local axis u₆ and orientable around the local axis u₆ in relation to platform 4 by means of a sixth actuator 18 (also preferably an electric motor). The local axis u₆ passes through an eccentric point Oₑ in relation to the platform centre Oₘ and when platform 4 stands in the neutral position, the local axis u₆ is parallel to the first axis e_{x,W} and is spaced from the platform centre Oₘ in the direction of the third axis e_{z,W}.

This introduces the sixth controllable degree of freedom (in series with respect to the five arms described above) which allows a rotation of the head with a twist of the neck close to the neck itself, while the remaining parts of device 1 can be kept away from_the head and easily placed beneath the operating table 3.

In accordance with an embodiment, with platform 4 in the neutral position and, preferably, in the entire positioning range ("range of motion") of platform 4, the first L_{1,1} and second L_{2,1} rods are transverse relative to each other (or, in other words, non-parallel) and positioned in such a way that, by adjusting the position of the first ends of the first L_{1,1} and second L_{2,1} rods along the first u_{1,1} and second u_{2,1} trajectories it is possible to move the position of the platform centre Oₘ in the direction of the first axis e_{x,W} and in the direction of the second axis e_{y,W}.
More specifically, with platform 4 in the neutral position and, preferably, in the entire positioning range ("range of motion") of platform 4, the orthogonal projections of longitudinal axes of the first L_{1,1} and second L_{2,1}rods on a plane (horizontal) defined by the first axis e_{X,W} and by the second axis e_{y,W} are mutually transversal (or, in other words, said projections are non-parallel) and, preferably, they intersect in the platform centre Oₘ in such a manner that, by adjusting the position of the first end of the first L_{1,1} and second L_{2,1} rods along the first u_{1,1} and second u_{2,1} trajectories it is possible to translate the position of the platform centre Oₘ in the direction of the first axis e_{X,W} and in the direction of the second axis e_{y,W}.

In other words, platform 4 and the first L_{1,1} and second L_{2,1} rods form a first articulated sub-frame 24, preferably an articulated trilateral frame, movable by means of the first 9 and second 11 actuators in such a way as to translate the position of the platform centre Oₘ in the direction of the first axis e_{x,W} and in the direction of the second axis e_{y,W}.

Likewise, with platform 4 in the neutral position and, preferably, in the entire positioning range ("range of motion") of platform 4, the third rod L₃ and the first L_{1,1} and second L_{2,1} rods are mutually transversal (or, in other words, are non-parallel) in such a manner that, by adjusting the position of the first end of the third rod L₃ and of both or only one of the first L_{1,1} and second L_{2,1} rods along the respective trajectories u_{3,1}, u_{1,1}, u_{2,1}, it is possible to translate the position of the platform centre Oₘ in the direction of the first axis e_{X,W} and in direction of the third axis e_{z,W}.
More specifically, with platform 4 in the neutral position and, preferably, in the entire positioning range ("range of motion") of platform 4, the orthogonal projection of a longitudinal axis of the third rod L₃ and of the longitudinal axis of at least one of the first L_{1,1} and second L_{2,1} rods (preferably of both) on a plane (longitudinal vertical) defined by the first axis e_{X,W} and by the third axis e_{z,W} are mutually transversal (or, in other words, said projections are non-parallel) and, possibly but not necessarily, they intersect in the platform centre Oₘ, in such a manner that, by adjusting the position of the first end of the third rod L₃ and of both or only one of the first L_{1,1} and second L_{2,1} rods along the respective trajectories u_{3,1}, u_{1,1}, u_{2,1}, it is possible to translate the position of the platform centre Oₘ in the direction of the first axis e_{X,W} and in the direction of the third axis e_{z,W}.

In other words, platform 4 and the third L₃, first L_{1,1} and/or second L_{2,1} rods form a second articulated sub-frame 25, for example an articulated trilateral or articulated quadrilateral frame, movable by means of the third 13, first 9 and/or second 11 actuators in such a way as to translate the position of the platform centre Oₘ in the direction of the first axis e_{x,W} and in the direction of the second axis e_{y,W.}

Furthermore, with platform 4 in the neutral position and, preferably, in the entire positioning range ("range of motion") of platform 4, the longitudinal axes of the fourth L₄ and fifth L₅ rods are parallel and spaced apart or they intersect at a point spaced from the platform centre Oₘ, in such a way that, by adjusting the position of the first ends of the fourth L₄ and fifth L₅ rods along the fourth u_{4,1} and fifth u_{5,1} trajectories, you can rotate the platform centre Oₘ (and thus the entire platform 4) around the third axis e_{z,W}.
More specifically, with platform 4 in the neutral position and, preferably, in the entire positioning interval ("range of motion") of platform 4, the orthogonal projections of longitudinal axes of the fourth L₄ and fifth L₅ rods on a plane (horizontal) defined by the first axis e_{X,W} and by the second axis e_{y,W} are parallel and spaced apart or they intersect in a point spaced from the platform centre Oₘ, in such a manner that, by adjusting the position of the first ends of the fourth L₄ and fifth L₅ rods along the fourth u_{4,1} and fifth u_{5,1} trajectories, it is possible to rotate the platform centre Oₘ (and therefore the entire platform 4) around the third axis e_{z,W}.

In other words, platform 4 and the fourth L₄ and fifth L₅ rods form a third articulated sub-frame 26, in particular an articulated quadrilateral frame, displaceable by means of the fourth 15 and fifth 17 actuators so as to rotate platform 4 around the third axis e_{z,W}.

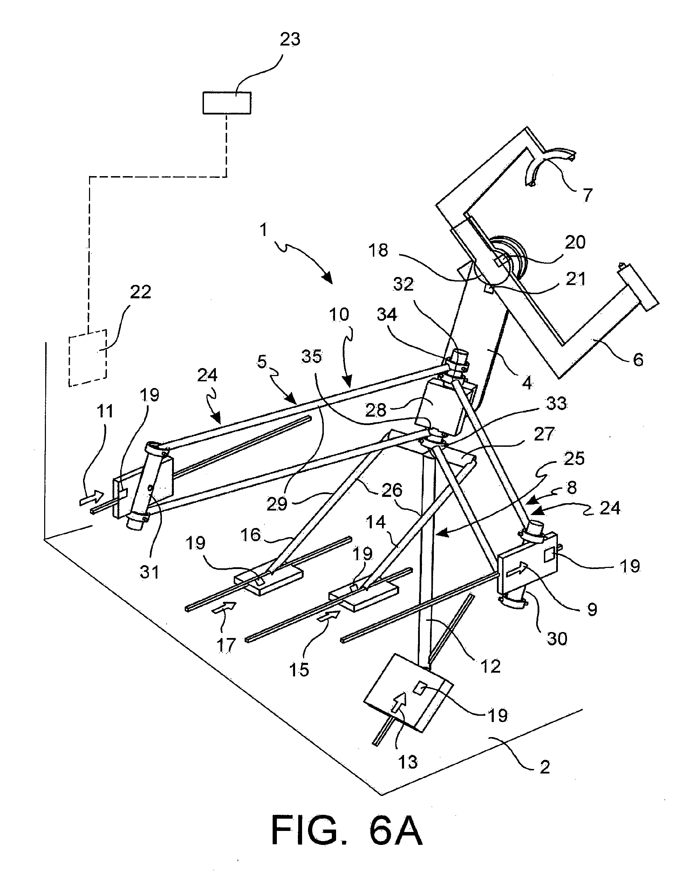

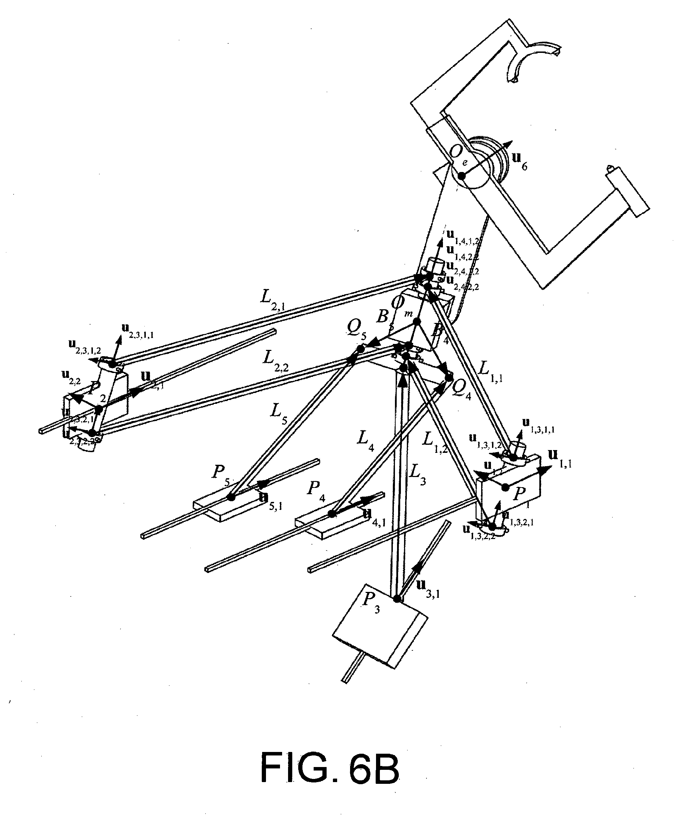

In accordance with an embodiment (Figures 6A, 6B), platform 4 has a T-shape with a longitudinal portion 28 which forms the platform centre Oₘ and a transversal portion 27 extending transversely with respect to the extension of the longitudinal portion. The transversal portion 27 forms two opposite ends spaced from the platform centre Oₘ with platform 4 in the neutral position, both in the direction of the second axis e_{y,W} and in the direction of the third axis e_{z,W}.

To form the third articulated sub-frame 26, the second end of the fourth rod L₄ and the second end of the fifth rod L₅ can be connected in a rotatable manner to one of the opposite ends of the transversal portion 27, respectively.

Moreover, with platform 4 in the neutral position and, preferably, in the entire positioning range ("range of motion") of platform 4, the longitudinal axis of at least one of the first L_{1,1} and second L_{2,1} rods (preferably of both) and of at least one of the fourth L₄ and fifth L₅ rods (preferably of both) are oriented and spaced apart, in such a manner that, by adjusting the position of the first end of the first L_{1,1} and/or second L_{2,1} and fourth L₄ and/or fifth L₅ rods along the respective trajectories u_{1,1}, u_{2,1}, u_{4,1} u_{5,1}, it is possible to rotate the platform centre Oₘ (and therefore the entire platform 4) around the second axis e_{y,W.}
More specifically, with platform 4 in the neutral position and, preferably, in the entire positioning range ("range of motion") of platform 4, the orthogonal projection of the longitudinal axis of at least one of the first L_{1,1} and second L_{2,1} rods (preferably of both) and of at least one of the fourth L₄ and fifth L₅ rods (preferably of both) on the plane (longitudinal vertical) defined by the first axis e_{X,W} and by the third axis e_{z,W} are parallel and spaced apart or they intersect in a point spaced from the platform centre Oₘ, in such a manner that, by adjusting the position of the first end of the first L_{1,1} and/or second L_{2,1} and fourth L₄ and/or fifth L₅ rods along the respective trajectories u_{1,1}, u_{2,1}, u_{4,1}, u_{5,1}, it is possible to rotate the platform centre Oₘ (and therefore the entire platform 4) around the second axis e_{y,W}.

In other words, at least one of the first L_{1,1} and second L_{2,1} rods and at least one of the fourth L₄ and fifth L₅ rod form, together with platform 4, at least a fourth articulated sub-frame 29, in particular an articulated quadrilateral frame, displaceable by means of the first 9 and/or second 11 and fourth 15 and/or or fifth 17 actuators so as to rotate platform 4 around the second axis e_{y,W.}

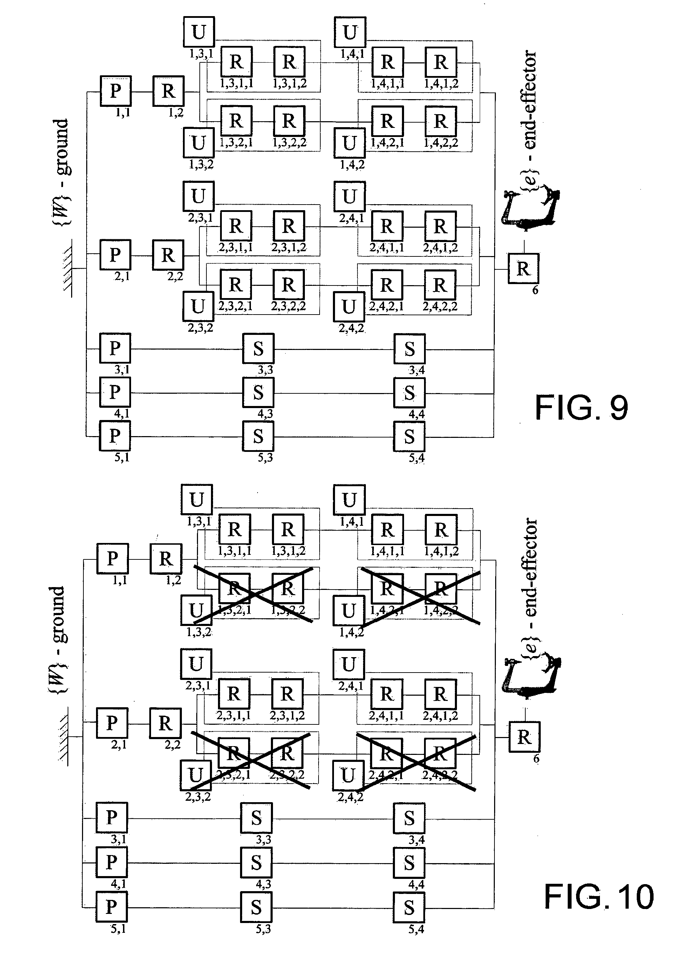

The topological representations of the adjusting mechanism 5 in Figures 9 to 14 show the five first 8, second 10, third 12, fourth 14 and fifth 16 kinematic arms with their joints and indicate:
prismatic joints (which only allow a rectilinear or also curvilinear translation) with the symbol "P";
simple rotary joints (which allow a rotation around a single axis of rotation) with the symbol "R";
universal or Cardan rotary joints (which allow a rotation around only two axes of rotation orthogonal to each other) with the symbol "U";
spherical rotary joints (which allow a spherical rotation around a polar centre of rotation) with the symbol "S".

In accordance with an embodiment (Figure 10) the first end of the first rod L_{1,1} is connected to the first saddle P₁ by a rotary or universal Cardan joint which allows the rotation around two axes u_{1,3,1,1} and u_{1,3,1,2} mutually orthogonal, mutually positioned in series, and orthogonal to the longitudinal axis of the first rod L_{1,1}, configured to transmit torsional moments to the first rod L_{1,1} and
the second end of the first rod L_{1,1} is connected to platform 4 by means of a rotary or universal Cardan joint which allows the rotation around two axes u_{1,4,1,1} and u_{1,4,1,2} mutually orthogonal and orthogonal to the longitudinal axis of the first rod L_{1,1}, reciprocally positioned in series, configured to transmit torsional moments to the first rod L_{1,1}.

Likewise, the first end of the second rod L_{2,1} is connected to the second saddle P₂ by a rotary or universal Cardan joint which allows the rotation around two axes u_{2,3,1,1} and u_{2,3,1,2} orthogonal to each other and orthogonal to the longitudinal axis of the second rod L_{2,1}, reciprocally positioned in series, configured to transmit torsional moments to the second rod L_{2,1}, and
the second end of the second rod L_{2,1} is connected to platform 4 by means of a rotary or universal Cardan joint which allows the rotation around two axes u_{2,4,1,1} and u_{2,4,1,2} orthogonal to each other and orthogonal to the longitudinal axis of the second rod L_{2,1}, reciprocally positioned in series, configured to transmit torsional moments to the second rod L_{2,1}.

In this way, it is possible to transmit torques around the first axis e_{x,W} from platform 4 to the supporting structure 2 by torsion of the first L_{1,1} and second L_{2,1} rods.

According to an alternative embodiment (Figure 9 and 6A, 6B), the first arm 8 includes a first leverage 30 connected to the first saddle P₁ by means of a simple rotary joint which allows the rotation around a single axis u_{1,2} orthogonal to the first path u_{1,1} and orthogonal to a longitudinal axis of the first leverage 30,
wherein the first end of the first rod L_{1,1} is connected to a first end of the first leverage 30 by means of a rotary or universal Cardan joint which allows a rotation around two axes orthogonal to each other, one of which u_{1,3,1,1} coaxial to the longitudinal axis of the first leverage 30 and the other u_{1,3,1,2} orthogonal to the longitudinal axis of the first rod L_{1,1},
and wherein a first end of a further first rod L_{1,2} is connected to a second end of the first leverage 30 (at a fixed distance from the first end of the first leverage 30) by means of a rotary or universal Cardan joint which allows a rotation around two axes orthogonal to each other, one of which u_{1,3,2,1} coaxial to the longitudinal axis of the first leverage 30 and the other u_{1,3,2,2} orthogonal to the longitudinal axis of the further first rod L_{1,2},
and wherein the second end of the first rod L_{1,1} is connected to platform 4 in a first connection point 32 via a Cardan or universal rotary joint which allows the rotation around two axes u_{1,4,1,1}, u_{1,4,1,2} orthogonal to each other, one of which u_{1,4,1,2} coaxial to a longitudinal axis of the longitudinal portion 28 of platform 4 and the other u_{1,4,1,1} orthogonal to the longitudinal axis of the first rod L_{1.1},
and in which a second end of the further first rod L_{1,2} is connected to platform 4 in a second connection point 33 (having fixed distance from the first connection point along the longitudinal axis of the longitudinal portion 28 of platform 4 and preferably identical to the distance between the first ends of the first rod L_{1,1} and of the further first rod L_{1,2}) by means of universal or Cardan rotary joint which allows the rotation around two axes u_{1,4,2,1}, u_{1,4,2,2} mutually orthogonal of which one u_{1,4,2,2} coaxial to a longitudinal axis of the longitudinal portion 28 of platform 4 and the other u_{1,4,2,1} orthogonal to the longitudinal axis of the further first rod L_{1,2}.

In this way, the first arm 8 forms an articulated quadrilateral linkage suitable to transmit, in certain positions, also the torques around the first axis e_{x,W} by axial forces in the first rod L_{1,1} and in the further first rod L_{1,2} spaced in the direction of the longitudinal axis of the first leverage 30 and, therefore, also in the direction of the third axis e_{z,W}.

Likewise, the second arm 10 comprises a second leverage 31 connected to the second saddle P₂ by means of a simple rotary joint which allows the rotation around a single axis u_{2,2} orthogonal to the second trajectory u_{2,1} and orthogonal to a longitudinal axis of the second leverage 31,
wherein the first end of the second rod L_{2,1} is connected to a first end of the second leverage 31 by means of a rotary or universal Cardan joint that allows a rotation around two axes orthogonal to each other, one of which u_{2,3,1,1} coaxial to the longitudinal axis of the second leverage 31 and the other u_{2,3,1,2} orthogonal to the longitudinal axis of the second rod L_{2,1},
and wherein a first end of a further second rod L_{2,2} is connected to a second end of the second leverage 31 (at a fixed distance from the first end of the second leverage 31) by means of a rotary or universal Cardan joint that allows a rotation around two axes orthogonal to each other, one of which u_{2,3,2,1} coaxial to the longitudinal axis of the second leverage 31 and the other u_{2,3,2,2} orthogonal to the longitudinal axis of the further second rod L_{2,2},
and wherein the second end of the second rod L_{2,1} is connected to platform 4 in a third connection point 34 (preferably close to or coinciding with the first connection point 32) by a rotary or universal Cardan joint which allows the rotation around two axes u_{2,4,1,1,} u_{2,4,1,2} orthogonal to each other, one of which u_{2,4,1,1} coaxial to the longitudinal axis of the longitudinal portion 28 of platform 4 and the other u_{2,4,1,2} orthogonal to the longitudinal axis of the second rod L_{2,1},
and wherein a second end of the further second rod L_{2,2} is connected to platform 4 in a fourth connection point 35 (having fixed distance from the third connection point 34 along the longitudinal axis of the longitudinal portion 28 of platform 4 and preferably identical to the distance between the first ends of the second rod L_{2,1} and of the further second rod L_{2,2}) by means of a universal or Cardan rotary joint that allows the rotation around two axes u_{2,4,2,1}, u_{2,4,2,2} mutually orthogonal of which one u_{2,4,2,1} coaxial to the longitudinal axis of the longitudinal portion 28 of platform 4 and the other u_{2,4,2,2} orthogonal to the longitudinal axis of the further second rod L_{2,2}.

The fourth connection point 35 may advantageously be immediately adjacent to or substantially coincident with the second connection point 33.

In this way, also the second arm 10 forms an articulated quadrilateral linkage suitable to transmit, in certain positions, the torques around the first axis e_{x,W} by axial forces in the second rod L_{2,1} and in the further second rod L_{2,2} spaced in the direction of the longitudinal axis of the second leverage 31 and, therefore, also in the direction of the third axis e_{z,W}.

The first end of the third rod L₃ can be connected to the third saddle P₃ using a spherical rotary joint or (to lock a rotation of the third rod around its longitudinal axis) a universal or Cardan rotary joint which allows the rotation around two axes orthogonal to each other and orthogonal to the longitudinal axis of the third rod L₃, and
the second end of the third rod L₃ is connected to platform 4 (preferably in the platform centre Oₘ) through a spherical rotary joint or a Cardan joint which allows the rotation around two axes orthogonal to each other and orthogonal to the longitudinal axis of the third rod L₃.

Also the first end of the fourth rod L₄ can be connected to the fourth saddle P₄ using a spherical rotary joint or, to block a rotation of the fourth rod around its longitudinal axis, by a universal or Cardan rotary joint which allows the rotation around two axes orthogonal to each other and orthogonal to the longitudinal axis of the fourth rod L₄, and
the second end of the fourth rod L₄ is connected to platform 4 by means of a spherical rotary joint or by a Cardan joint which allows the rotation around two axes orthogonal to each other and orthogonal to the longitudinal axis of the fourth rod L₄. Generally, in the case of a rod with two spherical joints at the ends, one of them can be replaced with a Cardan joint, to lock the rotation of the rod around its axis, keeping the second spherical joint to ensure the degrees of rotating freedom indispensable for proper functioning.

Finally, also the first end of the fifth rod L₅ can be connected to the fifth saddle P₅ by a spherical rotary joint or, to lock a rotation of the fifth rod around its longitudinal axis, by means of a universal or Cardan rotary joint which allows the rotation around two axes orthogonal to each other and orthogonal to the longitudinal axis of the fifth rod L₅, and
the second end of the fifth rod L₅ is connected to platform 4 by a spherical rotary joint or by a Cardan joint which allows the rotation around two axes orthogonal to each other and orthogonal to the longitudinal axis of the fifth rod L₅.

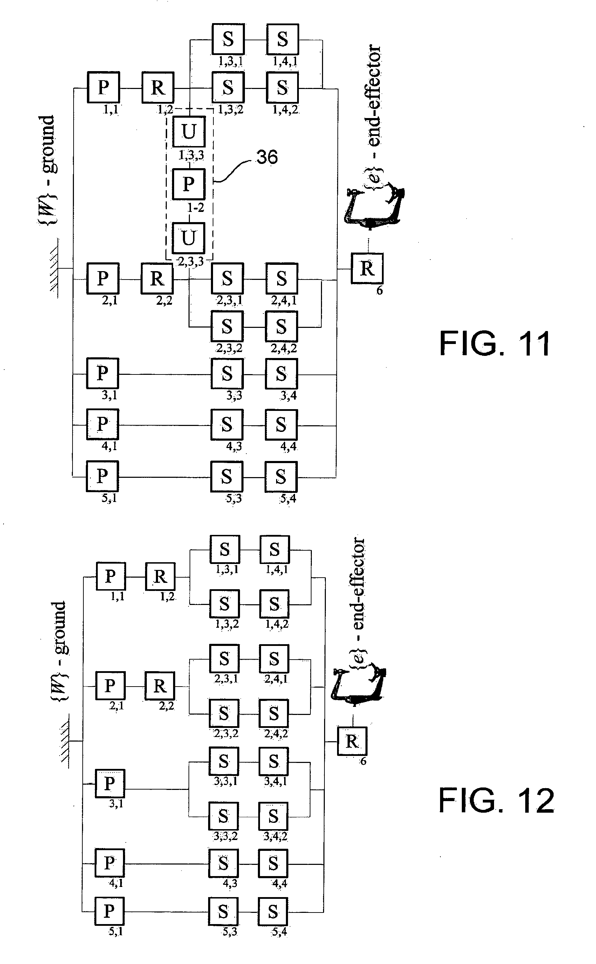

According to an alternative embodiment (Figure 11), it is possible to replace the Cardan joints of the first arm 8 and the second arm 10 with spherical joints, preventing the respective rods L_{1,1}, L_{1,2}, L_{2,1}, L_{2,2} to transmit torsional moments. In any case, to ensure the transmission of moments around the first axis e_{x,W} from platform 4 to the base structure 2, the first leverage 30 and the second leverage 31 may be coupled to each other by means of a double Cardan (constant velocity) joint 36 with variable length, so as to maintain the longitudinal axes of the first leverage 30 and the second leverage 31 always parallel.

As an alternative to the double Cardan constant velocity joint 36 and in any case to ensure the transmission of moments around the first axis e_{x,W} from platform 4 to the base structure 2, the third arm 12 may comprise a further third rod and form an articulated quadrilateral linkage suitable to transmit the moments around the first axis e_{x,W} from platform 4 to the base structure 2 by means of axial forces in the third rod and in the further third rod (Figure 12).

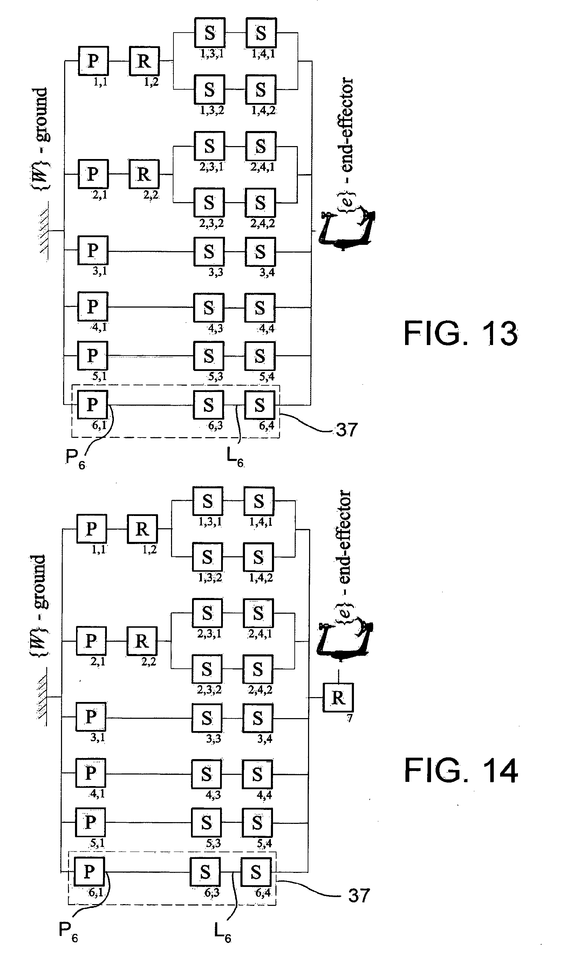

According to an embodiment with six parallel degrees of freedom (Figures 13, 14), a sixth arm 37 may be provided, having a sixth saddle P₆ slidably connected to the supporting structure 2 along a sixth trajectory, and positionable along the sixth trajectory by a sixth actuator, as well as a sixth rigid rod having a first end rotatably connected to the sixth saddle P₆ and a second end rotatably connected to platform 4.

With platform 4 in the neutral position and, preferably, in the entire positioning range ("range of motion") of platform 4, the longitudinal axes of the third L₃ and sixth L₆ rods are parallel and spaced apart or they intersect at a point spaced from the platform centre Oₘ, in such a way that, by adjusting the position of the first ends of the third L₃ and sixth L₆ rods along the third u_{3,1} and sixth trajectories, it is possible to rotate the platform centre Oₘ (and thus the entire platform 4) around the first axis e_{x,W}.
More specifically, with platform 4 in the neutral position and, preferably, in the entire positioning range ("range of motion") of platform 4, the orthogonal projections of longitudinal axes of the third L₃ and sixth L₆ rods on a plane (transversal vertical) defined by the second axis e_{y,W} and by the third axis e_{z,W} are parallel and spaced apart or they intersect in a point spaced from the platform centre Oₘ, in such manner that, by adjusting the position of the first end of the third L₃ and sixth L₆ rods along the third u_{4,1} and sixth trajectories, it is possible to rotate the platform centre Oₘ (and therefore the entire platform 4) around the first axis e_{X,W}.

In other words, platform 4 and the third L₃ and sixth L₆ rods form a further articulated sub-frame, in particular an articulated quadrilateral frame, displaceable by means of the third 13 and sixth actuators in such a way as to rotate platform 4 around the first axis e_{x,W}.

In order to form the further articulated sub-frame, the second end of the third rod L₃ and the second end of the sixth rod L₆ can be connected in a rotatable manner to one of the opposite ends of the transversal portion 27 or of another portion of platform 4 spaced from the transversal portion 27, respectively.

According to an embodiment, device 1 comprises sensor means associated to the adjusting mechanism 5, preferably saddle position sensors 19 located and configured to detect the positions of the first, second, third, fourth, and fifth (and, if provided, sixth) saddles in relation to the supporting structure 2, a gripping position sensor 20 arranged and configured to detect the rotary position of the gripping device 6 in relation to platform 4, as well as a force sensor 21 arranged and configured to detect the forces transmitted by the gripping device 6 to platform 4.

Device 1 further comprises a control unit 22 connected with the sensor means 19, 20, 21 and the actuators 9, 11, 13, 15, 17, 18, wherein the control unit 22 is configured to actuate the first, second, third, fourth, fifth, and sixth actuators so as to adjust the position of platform 4 and the gripping device 6 as a function of the amounts detected by the sensor means 19, 20, 21 of a control program and adjustment commands that can be received by a user interface 23.

The control program can be standardized or custom, loaded on a memory media queried by the control unit 22 and adapted in accordance with the demands of surgery planned.

Actuators 9, 11, 13, 15, 17, 18 can be driven using a control method based on the kinematic calculation for parallel and hybrid kinematic mechanisms. The control method per se does not form an object of the present invention.

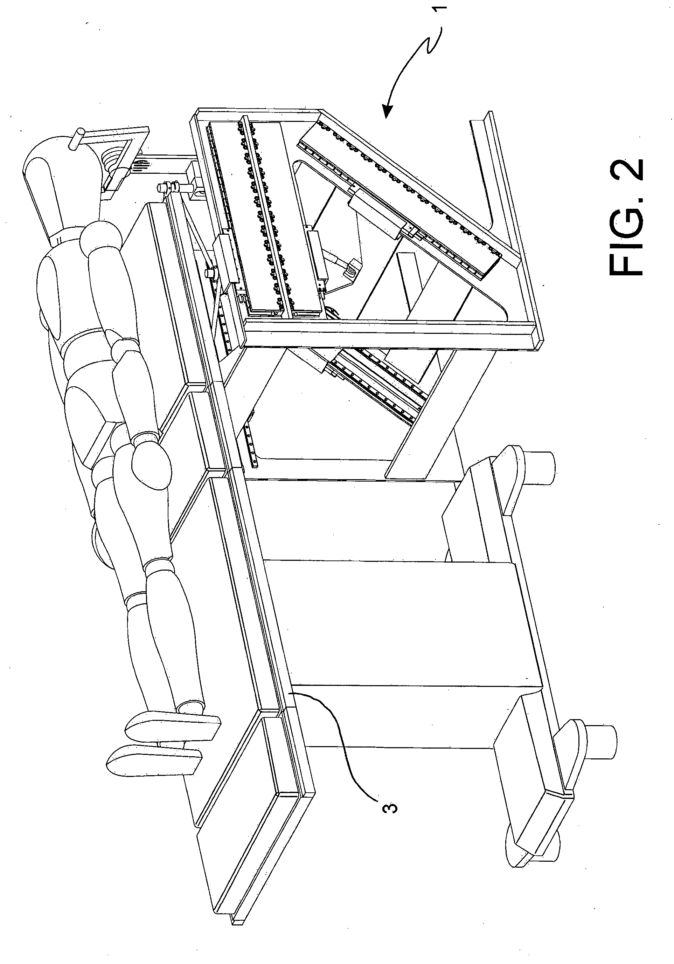

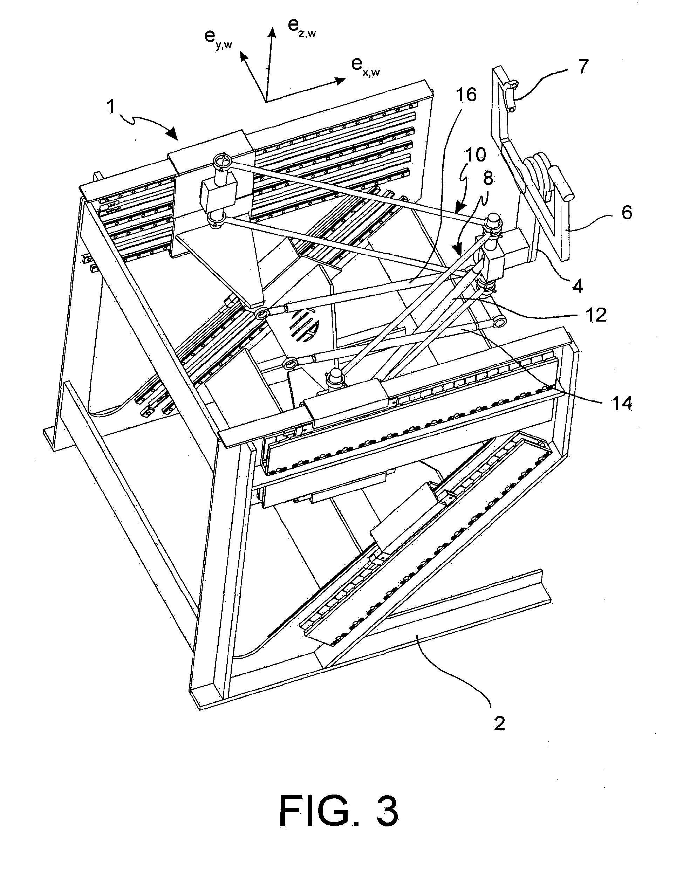

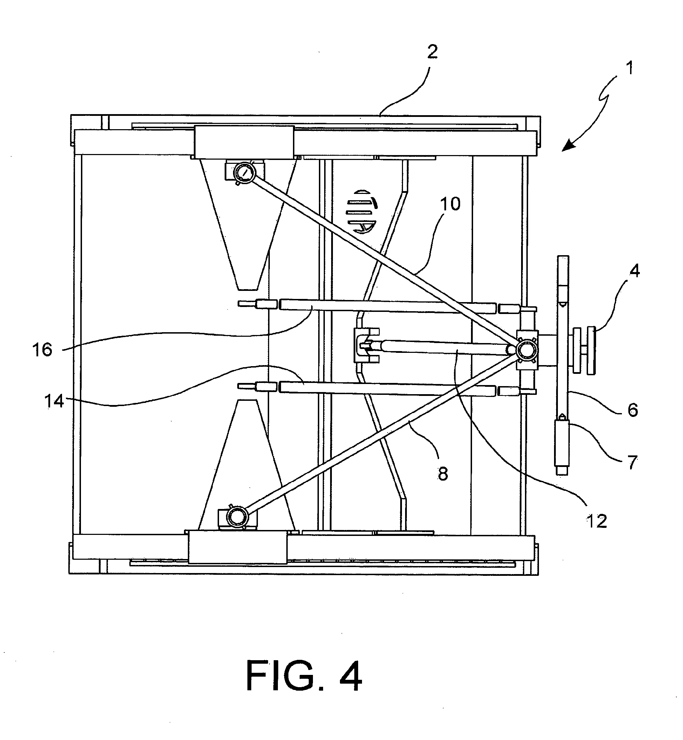

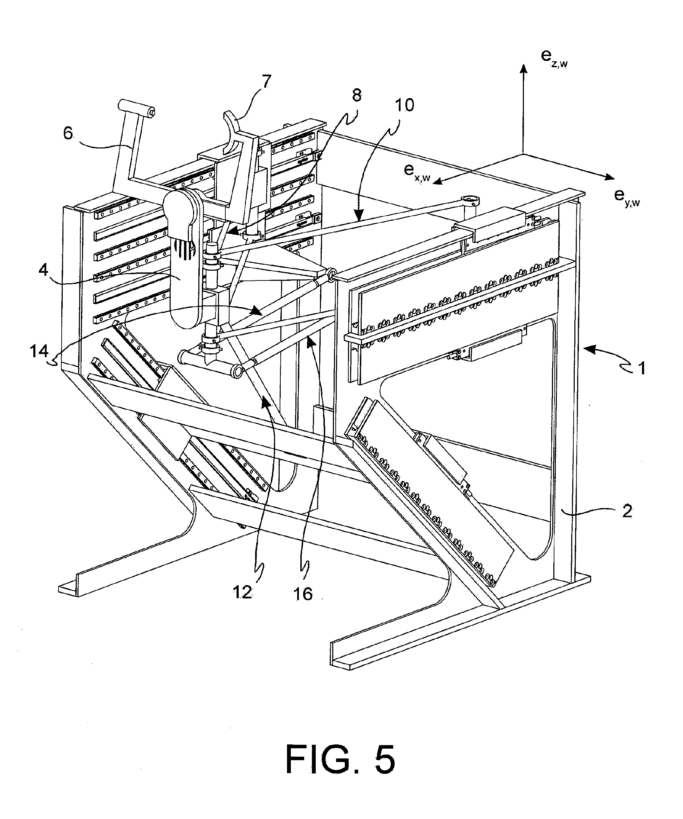

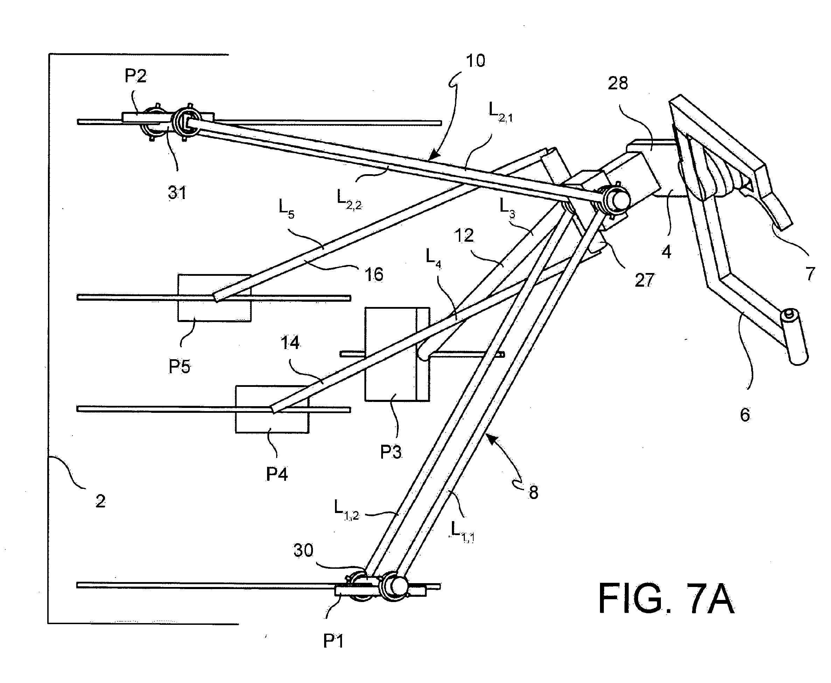

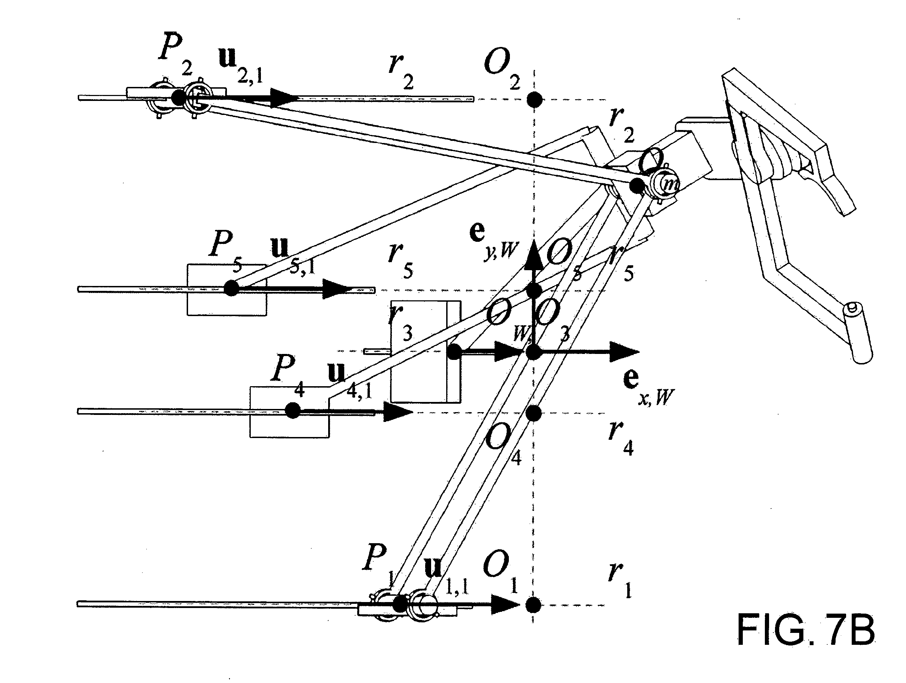

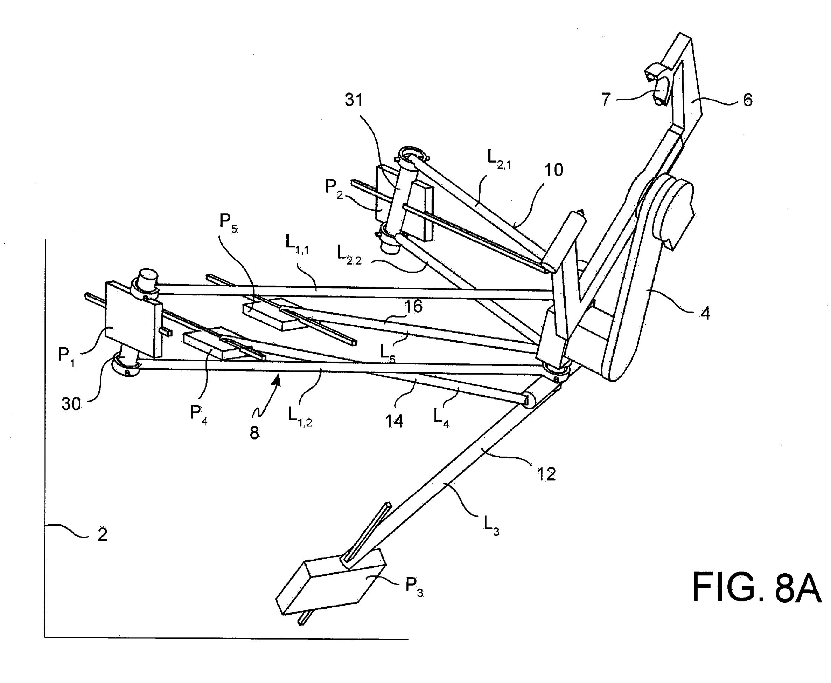

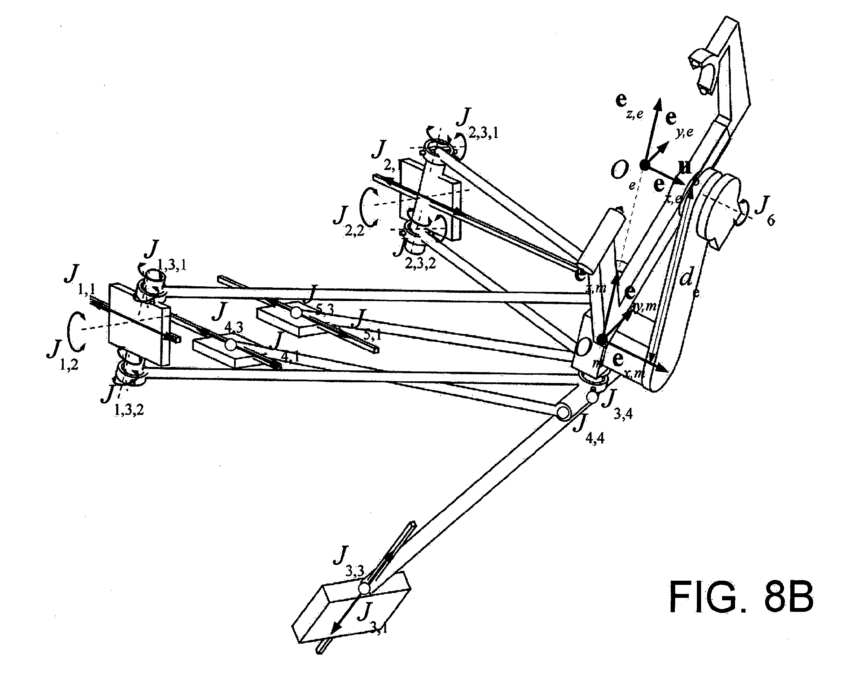

According to an embodiment (figures 2 to 8B), to facilitate the installation of the device in the available space beneath the surgery bed and the position control of platform 4, the first u_{1,1}, second u_{2,1}, third u_{3,1}, fourth u_{4,1} and fifth u_{5,1} trajectories are rectilinear. Advantageously, the first u_{1,1} and second u_{2,1} trajectories are parallel to each other and, preferably, parallel to the first axis e_{x,W}. With further advantage, also the fourth u_{4,1} and fifth u_{5,1} trajectories are parallel to each other and, preferably, parallel to the first axis e_{x,W}. According to a preferred embodiment, all first u_{1,1}, second u_{2,1}, third u_{3,1}, fourth u_{4,1} and fifth u_{5,1} trajectories lie in planes parallel to each other and, preferably, in planes parallel to a plane (longitudinal-vertical) axes defined by the first e_{x,W} (longitudinal axis) and third e_{z,W} axes of the supporting structure 2.

The third trajectory u_{3,1} is advantageously inclined or perpendicular to a plane (horizontal) defined by the first e_{x,W} and second e_{y,W} axes.

The supporting structure 2 may also comprise adjustment means, for example height adjustable feet, for adjusting the relative position between the supporting structure 2 and a floor.

The supporting structure 2 may further comprise fastening means, such as a fixing bracket with tightening screws (not shown in the figures) for the rigid fixing of device 1 to an application, for example to the operating table 3.

The supporting structure 2, arms 8, 10, 12, 14, 16, platform 4 and the gripping device 6 may be made of metal, e.g. aluminium, or synthetic material optionally reinforced with fibres, as well as of a composite material, for example a carbon matrix reinforced with carbon fibres or glass. In this way it is possible to combine high rigidity with a low weight that favours the transportability of device 1.

The adjusting mechanism 5 is advantageously covered by a flexible film or through a folding fabric (not shown) connected to the supporting structure 2 and to platform 4.

From the above description, the man skilled in the art will appreciate that device 1 according to the present invention allows the achievement of the objects described in the introduction and not repeated herein for shortness.

## Claims

1. A device (1) for supporting and adjusting the position of a patient's head during surgeries, comprising:
- a supporting structure (2) for resting the device (1) on an application, said supporting structure (2) defining a first axis (e_{x,W}), a second axis (e_{y,W}), and a third axis (e_{z,W}) that are mutually orthogonal,
- a mobile platform (4) defining a platform centre (Oₘ) and being constrained to the supporting structure (2) by an adjusting mechanism (5) suitable to position the platform (4) in relation to the supporting structure (2) in a neutral position and to adjust the position of the platform (4) in relation to the supporting structure (2).
- a gripping device (6) connected to the platform (4) and having locking means (7) suitable to lock the head to the gripping device (6),
**characterized in that** the adjusting mechanism (5) comprises:
- a first arm (8) having a first saddle (P₁) slidably connected to the supporting structure (2) along a first trajectory (u_{1,1}) and positionable along the first trajectory (u_{1,1}) by a first actuator (9), as well as a first rod (L_{1,1}) having a first end rotatably connected to the first saddle (P₁) and a second end rotatably connected to the platform (4),
- a second arm (10) having a second saddle (P₂) slidably connected to the supporting structure (2) along a second trajectory (u_{2,1}) and positionable along the second trajectory (u_{2,1}) by a second actuator (11), as well as a second rod (L_{2,1}) having a first end rotatably connected to the second saddle (P₂) and a second end rotatably connected to the platform (4),
- a third arm (12) having a third saddle (P₃) slidably connected to the supporting structure (2) along a third trajectory (u_{3,1}) and positionable along the third trajectory (u_{3,1}) by a third actuator (13), as well as a third rod (L3) having a first end rotatably connected to the third saddle (P₃) and a second end rotatably connected to the platform (4),
- a fourth arm (14) having a fourth saddle (P₄) slidably connected to the supporting structure (2) along a fourth trajectory (u_{4,1}) and positionable along the fourth trajectory (u_{4,1}) by a fourth actuator (15), as well as a fourth rod (L₄) having a first end rotatably connected to the fourth saddle (P₄) and a second end rotatably connected to the platform (4),
- a fifth arm (16) having a fifth saddle (P₅) slidably connected to the supporting structure (2) along a fifth trajectory (u_{5,1}) and positionable along the fifth trajectory (u_{5,1}) by a fifth actuator (17), as well as a fifth rod (L_{5,1}) having a first end rotatably connected to the fifth saddle (P₅) and a second end rotatably connected to the platform (4),
- wherein the platform (4) and the first (8), second (10), third (12), fourth (14), and fifth (16) arms form together an articulated frame displaceable by the first (9), second (11), third (13), fourth (15), and fifth (17) actuators so that the platform (4) can translate in the direction of the first axis (e_{x,W}), the second axis (e_{y,W}), and the third axis (e_{z,W}) and rotate around the second axis (e_{y,W}) and the third axis (_{z,W}).

2. The device (1) according to claim 1, wherein the first (9), second (11), third (13), fourth (15), and fifth (17) actuators comprise electric motors connected to the supporting structure (2) and acting on the first ends of the first (L_{1,1}), second (L_{2,1}), third (L₃), fourth (L₄), and fifth (L₅) rods.

3. The device (1) according to claim 1 or 2, wherein the gripping device (6) is rotatably connected to the platform (4) around a local axis (u₆) and orientable around the local axis (u₆) in relation to the platform (4) by a sixth actuator (18) connected to the platform (4).

4. The device (1) according to claim 3, wherein the local axis (u6) passes through a point (Oₑ) that is eccentric relative to the platform centre (Oₘ), and when the platform (4) stands in the neutral position, the local axis (u₆) is parallel to the first axis (e_{x,W}) and is spaced apart from the platform centre (Om) in the direction of the third axis (e_{z,W}).

5. The device (1) according to any one of the previous claims, wherein, within the entire positioning range of the platform (4), the longitudinal axes of the first (L_{1,1}) and the second (L_{2,1}) rods are transversal to one another, so that, by adjusting the position of the first ends of the first (L_{1,1}) and the second (L_{2,1}) rods along the first (u_{1,1}) and the second (u_{2,1}) trajectories, it is possible to translate the platform (4) in the direction of the first axis (e_{x,W}) and in the direction of the second axis (e_{y,W}).

6. The device (1) according to any one of the previous claims, wherein the platform (4) and the first (L_{1,1}) and the second (L_{2,1}) rods form a first articulated sub-frame (24) displaceable by the first (9) and the second (11) actuators so as to translate the platform (Oₘ) in the direction of the first axis (e_{x,W}) and in the direction of the second axis (e_{y,W}),
wherein the first articulated sub-frame (24) preferably forms an articulated trilateral linkage.

7. The device (1) according to any one of the previous claims, wherein the platform (4) and the third rod (L_{3,1}), and at least one of the first (L_{1,1}) and the second (L_{2,1}) rods, form a second articulated sub-frame (25) displaceable by the third (13), first (9) and/or second (11) actuators so as to translate the platform (4) in the direction of the first axis (e_{x,W}) and in the direction of the third axis (e_{z,W}).
wherein the second articulated sub-frame (25) preferably forms an articulated quadrilateral linkage.

8. The device (1) according to any one of the previous claims, wherein the longitudinal axes of the fourth (L4) and the fifth (L₅) rods are oriented and spaced apart, so that, by adjusting the position of the first ends of the fourth (L₄) and the fifth (L₅) rods along the fourth (u_{4,1}) and the fifth (u_{5,1}) trajectories, it is possible to rotate the platform (4) around the third axis (e_{z,W}).

9. The device (1) according to any one of the previous claims, wherein the platform (4) and the fourth (L₄) and the fifth (L₅) rods form a third articulated sub-frame (26) displaceable by the fourth (15) and the fifth (17) actuators so as to rotate the platform (4) around the third axis (e_{z,W}),
wherein the third articulated sub-frame (26) preferably forms an articulated quadrilateral linkage.

10. The device (1) according to claim 9, wherein the platform (4) is in the shape of a (T) with a longitudinal portion (28) forming the platform centre (Oₘ), and a transversal portion (27) transversally extending with respect to the extension of the longitudinal portion (28), wherein the transversal portion (27) forms two opposite ends spaced apart from the platform centre (Oₘ) both in the direction of the second axis (e_{y,W}) and in the direction of the third axis (e_{z,W}), wherein the second end of the fourth rod (L4) and the second end of the fifth rod (L₅) are rotatably connected respectively to one of the opposite ends of the transversal portion (27).

11. The device (1) according to any one of the previous claims, wherein, within the entire positioning range of the platform (4), the orthogonal projections of the longitudinal axis of at least one of the first (L_{1,1}) and the second (L_{2,1}) rods and of at least one of the fourth (L₄) and the fifth (L₅) rods on the plane defined by the first axis (e_{x,W}) and the third axis (e_{z,W}) are parallel and spaced apart or intersect in a point spaced apart from the platform centre (Oₘ), so that, by adjusting the position of the first ends of at least one of the first (L_{1,1}) and the second (L_{2,1}) rods and of at least one of the fourth (L₄) and the fifth (L₅) rods along the respective trajectories (u_{1,1}, u_{2,1}, u_{4,1}, u_{5,1}). it is possible to rotate the platform (4) around the second axis (e_{y,W}).

12. The device (1) according to any one of the previous claims, wherein at least one of the first (L_{1,1}) and the second (L_{2,1}) rods and at least one of the fourth (L₄) and the fifth (L₅) rods form, together with the platform (4), at least one fourth articulated sub-frame (29) displaceable by the respective actuators (9, 11, 15, 17) so as to rotate the platform (4) around the second axis (e_{y,W}), wherein the fourth sub-frame (29) forms an articulated quadrilateral linkage.

13. The device (1) according to any one of the previous claims, wherein:
the first rod (L_{1,1}) and the second rod (L_{2,1}) form, at the ends thereof, rotary Cardan joints that are configured to transmit torques to the rod, while allowing the rotation around two axes that are mutually orthogonal and orthogonal to a longitudinal axis of the respective rod.

14. The device (1) according to any one of the previous claims, wherein:
- the first arm (8) further comprises a first leverage (30) rotatably connected to the first saddle (P₁),
wherein the first end of the first rod (L_{1,1}) is rotatably connected to a first end of the first leverage (30), and a first end of a further first rod (L_{1,2}) is rotatably connected to a second end of the first leverage (30),
wherein the second end of the first rod (L_{1,1}) is connected to the platform (4) in a first connection point (32), and a second end of the further first rod (L_{1,2}) is connected to the platform (4) in a second connection point (33),
so that the first arm (8) and the platform (4) form an articulated quadrilateral linkage,
- the second arm (10) further comprises a second leverage (31) that is rotatably connected to the second saddle (P₂),
wherein the first end of the second rod (L_{2,1}) is rotatably connected to a first end of the second leverage (31), and a first end of a further second rod (L_{2,2}) is rotatably connected to a second end of the second leverage (31),
wherein the second end of the second rod (L_{2,1}) is connected to the platform (4) in a third connection point (34), and a second end of the further second rod (L_{2,2}) is rotatably connected to the platform (4) in a fourth connection point (35),
so that the second arm (10) and the platform form an articulated quadrilateral linkage,
wherein the third connection point (34) is immediately adjacent to the first connection point (32), and the fourth connection point (35) is immediately adjacent to the second connection point (33), and the first, second, third, and fourth connection points are all arranged on a longitudinal axis of the platform (4), allowing a rotation of the second ends of the first, second, third, and fourth rods in relation to the platform (4) around the platform (4) longitudinal axis,
wherein the first leverage (30) and the second leverage (31) are mutually coupled by a homokinetic double Cardan joint of a variable length (36) and configured so as to keep the longitudinal axes of the first leverage (30) and the second leverage (31) always parallel.

15. The device (1) according to one of the previous claims, comprising a sixth arm (37) having a sixth saddle (P₆) that is slidably connected to the supporting structure (2) along a sixth trajectory, and positionable along the sixth trajectory by a sixth actuator, as well as a sixth rigid rod having a first end rotatably connected to the sixth saddle (P₆), and a second end rotatably connected to the platform (4),
wherein, within the entire positioning range of the platform (4), the longitudinal axes of the third (L₃) and sixth (L₆) rods are oriented and spaced apart, so that, by adjusting the position of the first ends of the third (L₃) and sixth (L₆) rods along the third (u_{3,1}) and sixth trajectories, it is possible to rotate the platform (4) around the first axis (e_{x,W}).

## Patentansprüche

1. Vorrichtung (1) zum Tragen und Anpassen der Position des Kopfs eines Patienten während Operationen, umfassend:
- eine Tragestruktur (2) zum Abstützen der Vorrichtung (1) an einer Einrichtung, wobei die Tragestruktur (2) eine erste Achse (eₓ, _{w}), eine zweite Achse (e_{y}, _{w}) und eine dritte Achse (e_{z}, _{w}) definiert, welche zueinander orthogonal sind,
- eine mobile Plattform (4), welche eine Plattform-Mitte (Oₘ) definiert und an der Tragestruktur (2) durch einen Anpassungsmechanismus (5) gehalten ist, welcher geeignet ist, die Plattform (4) bezüglich der Tragestruktur (2) in einer neutralen Position zu positionieren und die Position der Plattform (4) bezüglich der Tragestruktur (2) anzupassen,
- eine Greifvorrichtung (6), welche mit der Plattform (4) verbunden ist und Fixierungsmittel (7) aufweist, welche geeignet sind, den Kopf an der Greifvorrichtung (6) zu fixieren,
**dadurch gekennzeichnet, dass** der Anpassungsmechanismus (5) umfasst:
- einen ersten Arm (8), welcher einen ersten Sattel (P₁) entlang einer ersten Bahn (u_{1,1}) gleitbar und durch einen ersten Aktuator (9) entlang der ersten Bahn (u_{1,1}) positionierbar mit der Tragestruktur (2) verbunden aufweist, sowie eine erste Stange (L₁,₁), welche ein erstes Ende rotierbar mit dem ersten Sattel (P₁) und ein zweites Ende rotierbar mit der Plattform (4) verbunden aufweist,
- einen zweiten Arm (10), welcher einen zweiten Sattel (P₂) entlang einer zweiten Bahn (u_{2,1}) gleitbar und durch einen zweiten Aktuator (11) entlang der zweiten Bahn (u_{2,1}) positionierbar mit der Tragestruktur (2) verbunden aufweist, sowie eine zweite Stange (L_{2,1}), welche ein erstes Ende rotierbar mit dem zweiten Sattel (P₂) und ein zweites Ende rotierbar mit der Plattform (4) verbunden aufweist,
- einen dritten Arm (12), welcher einen dritten Sattel (P₃) entlang einer dritten Bahn (u_{3,1}) gleitbar und durch einen dritten Aktuator (13) entlang der dritten Bahn (u_{3,1}) positionierbar mit der Tragestruktur (2) verbunden aufweist, sowie eine dritte Stange (L3), welche ein erstes Ende rotierbar mit dem dritten Sattel (P₃) und ein zweites Ende rotierbar mit der Plattform (4) verbunden aufweist,
- einen vierten Arm (14), welcher einen vierten Sattel (P₄) entlang einer vierten Bahn (u_{4,1}) gleitbar und durch einen vierten Aktuator (15) entlang der vierten Bahn (u_{4,1}) positionierbar mit der Tragestruktur (2) verbunden aufweist, sowie eine vierte Stange (L₄), welche ein erstes Ende rotierbar mit dem vierten Sattel (P₄) und ein zweites Ende rotierbar mit der Plattform (4) verbunden aufweist,
- einen fünften Arm (16), welcher einen fünften Sattel (P₅) entlang einer ersten Bahn (u_{5,1}) gleitbar und durch einen fünften Aktuator (17) entlang der fünften Bahn (u_{5,1}) positionierbar mit der Tragestruktur (2) verbunden aufweist, sowie eine fünfte Stange (L_{5,1}), welche ein erstes Ende rotierbar mit dem fünften Sattel (P₅) und ein zweites Ende rotierbar mit der Plattform (4) verbunden aufweist,
- wobei die Plattform (4) und der erste (8), zweite (10), dritte (12), vierte (14) und fünfte (16) Arm zusammen einen gelenkigen Rahmen bilden, welcher von dem ersten (9), zweiten (11), dritten (13), vierten (15) und fünften (17) Aktuator verstellbar ist, so dass sich die Plattform (4) in der Richtung der ersten Achse (e_{x,w}), der zweiten Achse (e_{y,w}) und der dritten Achse (e_{z,w}) verlagern und um die zweite Achse (e_{y,w}) und die dritte Achse (e_{z,w}) rotieren kann.

2. Vorrichtung (1) nach Anspruch 1, wobei der erste (9), zweite (11), dritte (13), vierte (15) und fünfte (17) Aktuator Elektromotoren umfassen, die mit der Tragestruktur (2) verbunden sind und auf die ersten Enden der ersten (L_{1,1}), zweiten (L_{2,1}), dritten (L₃), vierten (L₄) und fünften (L₅) Stange wirken.

3. Vorrichtung (1) nach Anspruch 1 oder 2, wobei die Greifvorrichtung (6) mit der Plattform (4) um eine lokale Achse (u₆) rotierbar verbunden ist und um die lokale Achse (u₆) bezüglich der Plattform (4) durch einen sechsten Aktuator (18) orientierbar ist, der mit der Plattform (4) verbunden ist.

4. Vorrichtung (1) nach Anspruch 3, wobei die lokale Achse (u6) durch einen Punkt (Oₑ) verläuft, der bezüglich der Plattform-Mitte (Oₘ exzentrisch ist, und wenn die Plattform (4) in der neutralen Position steht, die lokale Achse (u₆) parallel zu der ersten Achse (e_{x,w}) ist und von der Plattform-Mitte (Om) in der Richtung der dritten Achse (e_{z,w}) beabstandet ist.

5. Vorrichtung (1) nach einem der vorhergehenden Ansprüche, wobei innerhalb des gesamten Positionierungsbereichs der Plattform (4) die Längsachsen der ersten (L_{1,1}) und der zweiten (L_{2,1}) Stange transversal zueinander sind, so dass es durch Anpassen der Position der ersten Enden der ersten (L_{1,1}) und der zweiten (L_{2,1}) Stange entlang der ersten (u_{1,1}) und der zweiten (u_{2,1}) Bahn möglich ist, die Plattform (4) in die Richtung der ersten Achse (e_{x,w}) und in die Richtung der zweiten Achse (e_{y,w}) zu verlagern.

6. Vorrichtung (1) nach einem der vorhergehenden Ansprüche, wobei die Plattform (4) und die erste (L_{1,1}) und die zweite (L_{2,1}) Stange einen ersten gelenkigen Unterrahmen (24) bilden, welcher durch den ersten (9) und den zweiten (11) Aktuator verstellbar ist, um so die Plattform (Oₘ) in der Richtung der ersten Achse (e_{x,w}) und in der Richtung der zweiten Achse (e_{y,w}) zu verlagern,
wobei der erste gelenkige Unterrahmen (24) vorzugsweise eine gelenkige dreiseitige Verbindung bildet.

7. Vorrichtung (1) nach einem der vorhergehenden Ansprüche, wobei die Plattform (4) und die dritte Stange (L_{3,1}) und wenigstens eine aus der ersten (L_{1,1}) und der zweiten (L_{2,1}) Stange einen zweiten gelenkigen Unterrahmen (25) bilden, welcher durch den dritten (13), ersten (9) oder/und zweiten (11) Aktuator verlagerbar ist, um so die Plattform (4) in die Richtung der ersten Achse (e_{x,w}) und in die Richtung der dritten Achse (e_{z,w}) zu verlagern,
wobei der zweite gelenkige Unterrahmen (25) vorzugsweise eine gelenkige, vierseitige Verbindung bildet.

8. Vorrichtung (1) nach einem der vorhergehenden Ansprüche, wobei die Längsachse der vierten (L4) und der fünften (L₅) Stange derart orientiert und beabstandet sind, dass es durch Anpassen der Position der ersten Enden der vierten (L₄) und fünften (L₅) Stange entlang der vierten (u_{4,1}) und der fünften (u_{5,1}) Bahn möglich ist, die Plattform (4) um die dritte Achse (e_{z,w}) zu rotieren.

9. Vorrichtung (1) nach einem der vorhergehenden Ansprüche, wobei die Plattform (4) und die vierte (L₄) und die fünfte (L₅) Stange einen dritten gelenkigen Unterrahmen (26) bilden, welcher durch den vierten (15) und fünften (17) Aktuator verlagerbar ist, so dass die Plattform (4) um die dritte Achse (e_{z,w}) rotiert,
wobei der dritte gelenkige Unterrahmen (26) vorzugsweise eine gelenkige vierseitige Verbindung bildet.

10. Vorrichtung (1) nach Anspruch 9, wobei die Plattform (4) in der Form eines (T) mit einem longitudinalen Abschnitt (28), welcher die Plattform-Mitte (Oₘ) bildet, und mit einem transversalen Abschnitt (27) vorliegt, welcher sich transversal bezüglich der Verlängerung des longitudinalen Abschnitts (28) erstreckt, wobei der transversale Abschnitt (27) zwei gegenüberliegende Enden bildet, welche von der Plattform-Mitte (Oₘ) sowohl in der Richtung der zweiten Achse (e_{y,w}) als auch in der Richtung der dritten Achse (e_{z,w}) beabstandet sind, wobei das zweite Ende der vierten Stange (L4) und das zweite Ende der fünften Stange (L₅) jeweils rotierbar mit einem der gegenüberliegenden Enden des transversalen Abschnitts (27) verbunden sind.

11. Vorrichtung (1) nach einem der vorhergehenden Ansprüchen, wobei innerhalb des gesamten Positionierungsbereichs der Plattform (4) die orthogonalen Projektionen der Längsachse von wenigstens einer aus der ersten (L_{1,1}) und der zweiten (L_{2,1}) Stange und von wenigstens einer aus der vierten (L₄) und der fünften (L₅) Stange auf die durch die ersten Achse (e_{x,w}) und die dritten Achse (e_{z,w}) definierte Ebene parallel und beabstandet sind oder sich in einem Punkt von der Plattform-Mitte (Oₘ) beabstandet schneiden, so dass es durch Anpassen der Position der ersten Enden von wenigstens einer aus der ersten (L_{1,1}) und der zweiten (L_{2,1}) Stange und wenigstens einer aus der vierten (L₄) und der fünften (L₅) Stange entlang der jeweiligen Bahnen (u_{1,1}, u_{2,1}, u_{4,1}, u_{5,1}) möglich ist, die Plattform (4) um die zweite Achse (e_{y,w}) zu rotieren.

12. Vorrichtung (1) nach einem der vorhergehenden Ansprüche, wobei wenigstens eine aus der ersten (L_{1,1}) und der zweiten (L_{2,1}) Stange und wenigstens eine aus der vierten (L₄) und der fünften (L₅) Stange zusammen mit der Plattform (4) wenigstens einen vierten gelenkigen Unterrahmen (29) bilden, welcher durch jeweilige Aktuatoren (9, 11, 15, 17) verlagerbar ist, um so die Plattform (4) um die zweite Achse (e_{y,w}) zu rotieren, wobei der vierte Unterrahmen (29) eine gelenkige vierseitige Verbindung bildet.

13. Vorrichtung (1) nach einem der vorhergehenden Ansprüche, wobei:
die erste Stange (L_{1,1}) und die zweite Stange (L_{2,1}) an den Enden davon Rotations-Kardangelenke bilden, welche dazu eingerichtet sind, Drehmomente an die Stange zu übertragen, während die Rotation um zwei Achsen erlaubt wird, die gegenseitig orthogonal und orthogonal zu einer Längsachse der jeweiligen Stange sind.

14. Vorrichtung (1) nach einem der vorhergehenden Ansprüche, wobei:
- der erste Arm (8) ferner einen ersten Hebel (30) umfasst, welcher rotierbar mit dem ersten Sattel (P₁) verbunden ist,
wobei das erste Ende der ersten Stange (L_{1,1}) rotierbar mit einem ersten Ende des ersten Hebels (30) verbunden ist, und ein erstes Ende einer weiteren ersten Stange (L_{1,2}) rotierbar mit einem zweiten Ende des ersten Hebels (30) verbunden ist,
wobei das zweite Ende der ersten Stange (L_{1,1}) mit der Plattform (4) in einem ersten Verbindungspunkt (32) verbunden ist, und ein zweites Ende der weiteren ersten Stange (L_{1,2}) mit der Plattform (4) in einem zweiten Verbindungspunkt (33) verbunden ist,
so dass der erste Arm (8) und die Plattform (4) eine gelenkige vierseitige Verbindung bilden,
- der zweite Arm (10) ferner einen zweiten Hebel (31) umfasst, welcher rotierbar mit dem zweiten Sattel (P₂) verbunden ist,
wobei das erste Ende der zweiten Stange (L_{2,1}) rotierbar mit einem ersten Ende des zweiten Hebels (31) verbunden ist, und ein erstes Ende einer weiteren zweiten Stange (L_{2,2}) rotierbar mit einem zweiten Ende des zweiten Hebels (31) verbunden ist,
wobei das zweite Ende der zweiten Stange (L_{2,1}) mit der Plattform (4) in einem dritten Verbindungspunkt (34) verbunden ist, und ein zweites Ende der weiteren zweiten Stange (L_{2,2}) rotierbar mit der Plattform (4) in einem vierten Verbindungspunkt (35) verbunden ist,
so dass der zweite Arm (10) und die Plattform eine gelenkige vierseitige Verbindung bilden,
wobei der dritte Verbindungspunkt (34) unmittelbar benachbart zu dem ersten Verbindungspunkt (32) ist, und der vierte Verbindungspunkt (35) unmittelbar benachbart zu dem zweiten Verbindungspunkt (33) ist, und der erste, zweite, dritte und vierte Verbindungspunkt alle an einer Längsachse der Plattform (4) angeordnet sind, was eine Rotation der zweiten Enden der ersten, zweiten, dritten und vierten Stange bezüglich der Plattform (4) um die Längsachse der Plattform (4) erlaubt,
wobei der erste Hebel (30) und der zweite Hebel (31) gegenseitig durch ein homokinetisches Doppel-Kardangelenk von einer variablen Länge (36) gekoppelt sind und derart eingerichtet sind, dass die Längsachsen des ersten Hebels (30) und des zweitens Hebels (31) stets parallel gehalten werden.

15. Vorrichtung (1) nach einem der vorhergehenden Ansprüche, umfassend einen sechsten Arm (37), welcher einen sechsten Sattel (P₆) aufweist, welcher gleitbar mit der Tragestruktur (2) entlang einer sechsten Bahn verbunden ist und durch einen sechsten Aktuator entlang der sechsten Bahn positionierbar ist, sowie eine sechste starre Stange, welche ein erstes Ende rotierbar mit dem sechsten Sattel (P₆) verbunden aufweist und ein zweites Ende rotierbar mit der Plattform (4) verbunden aufweist,
wobei innerhalb des gesamten Positionierungsbereichs der Plattform (4) die Längsachsen der dritten (L₃) und sechsten (L₆) Stange derart orientiert und beabstandet sind, dass es durch Anpassen der Position der ersten Enden der dritten (L₃) und sechsten (L₆) Stange entlang der dritten (u_{3,1}) und sechsten Bahn möglich ist, die Plattform (4) um die erste Achse (e_{x,w}) zu rotieren.

## Revendications

1. Dispositif (1) pour supporter et ajuster la position de la tête d'un patient pendant des opérations chirurgicales, comprenant :
uns structure de support (2) pour appuyer le dispositif (1) sur une application, ladite structure de support (2) définissant un premier axe (e_{x,w}), un deuxième axe (e_{y,w}), et un troisième axe (e_{z,w}) qui sont mutuellement orthogonaux,
une plateforme mobile (4) définissant un centre de plateforme (Oₘ) et étant contrainte sur la structure de support (2) par un mécanisme d'ajustement (5) approprié pour positionner la plateforme (4) par rapport à la structure de support (2) dans une position neutre et pour ajuster la position de la plateforme (4) par rapport à la structure de support (2),
un dispositif de préhension (6) raccordé à la plateforme (4) et ayant des moyens de verrouillage (7) appropriés pour bloquer la tête sur le dispositif de préhension (6),
**caractérisé en ce que** le mécanisme d'ajustement (5) comprend :
un premier bras (8) ayant une première selle (P₁) raccordée de manière coulissante à la structure de support (2) le long d'une première trajectoire (u_{1,1}) et pouvant être positionnée le long de la première trajectoire (u_{1,1}) par un premier actionneur (9), ainsi qu'une première tige (L_{1,1}) ayant une première extrémité raccordée de manière rotative à la première selle (P₁) et une seconde extrémité raccordée de manière rotative à la plateforme (4),
un deuxième bras (10) ayant une deuxième selle (P₂) raccordée de manière coulissante à la structure de support (2) le long d'une deuxième trajectoire (u_{2,1}) et pouvant être positionnée le long de la deuxième trajectoire (_{U2,1}) par un deuxième actionneur (11), ainsi qu'une deuxième tige (L_{2,1}) ayant une première extrémité raccordée de manière rotative à la deuxième selle (P₂) et une deuxième extrémité raccordée de manière rotative à la plateforme (4),
un troisième bras (12) ayant une troisième selle (P₃) raccordée de manière coulissante à la structure de support (2) le long d'une troisième trajectoire (u_{3,1}) et pouvant être positionnée le long de la troisième trajectoire (u_{3,1}) par un troisième actionneur (13), ainsi qu'une troisième tige (L3) ayant une première extrémité raccordée de manière rotative à la troisième selle (P₃) et une seconde extrémité raccordée de manière rotative à la plateforme (4),
un quatrième bras (14) ayant une quatrième selle (P₄) raccordée de manière coulissante à la structure de support (2) le long d'une quatrième trajectoire (u_{4,1}) et pouvant être positionnée le long de la quatrième trajectoire (u_{4,1}) par un quatrième actionneur (15), ainsi qu'une quatrième tige (L₄) ayant une première extrémité raccordée de manière rotative à la quatrième selle (P₄) et une seconde extrémité raccordée de manière rotative à la plateforme (4),
un cinquième bras (16) ayant une cinquième selle (P₅) raccordée de manière coulissante à la structure de support (2) le long d'une cinquième trajectoire (u_{5,1}) et pouvant être positionnée le long de la cinquième trajectoire (u_{5,1}) par un cinquième actionneur (17) ainsi qu'une cinquième tige (L_{5,1}) ayant une première extrémité raccordée de manière rotative à la cinquième selle (P₅) et une seconde extrémité raccordée de manière rotative à la plateforme (4),
dans lequel la plateforme (4) et les premier (8), deuxième (10), troisième (12), quatrième (14) et cinquième (16) bras forment ensemble un châssis articulé déplaçable par les premier (9), deuxième (11), le troisième (13), quatrième (15) et cinquième (17) actionneurs de sorte que la plateforme (4) peut effectuer un mouvement de translation dans la direction du premier axe (e_{x,w}), du deuxième axe (e_{y,w}), et du troisième axe (e_{z,w}) et tourner autour du deuxième axe (e_{y,w}) et du troisième axe (e_{z,w}).

2. Dispositif (1) selon la revendication 1, dans lequel les premier (9), deuxième (11), troisième (13), quatrième (15) et cinquième (17) actionneurs comprennent des moteurs électriques raccordés à la structure de support (2) et agissant sur les premières extrémités des première (L_{1,1}), deuxième (L_{2,1}), troisième (L₃), quatrième (L₄) et cinquième (L₅) tiges.

3. Dispositif (1) selon la revendication 1 ou 2, dans lequel le dispositif de préhension (6) est raccordé de manière rotative à la plateforme (4) autour d'un axe local (u₆) et orientable autour de l'axe local (u₆) par rapport à la plateforme (4) par un sixième actionneur (18) raccordé à la plateforme (4).

4. Dispositif (1) selon la revendication 3, dans lequel l'axe local (u6) passe par un point (Oₑ) qui est excentrique par rapport au centre de plateforme (Oₘ), et lorsque la plateforme (4) est dans la position neutre, l'axe local (u₆) est parallèle au premier axe (e_{x,w}) et est éloigné du centre de plateforme (Om) dans la direction du troisième axe (e_{z,w}).

5. Dispositif (1) selon l'une quelconque des revendications précédentes, dans lequel, dans toute la plage de positionnement de la plateforme (4), les axes longitudinaux des première (L_{1,1}) et deuxième (L_{2,1}) tiges sont transversaux les uns par rapport aux autres, de sorte que, en ajustant la position des premières extrémités des première (L_{1,1}) et deuxième (L_{2,1}) tiges le long des première (u_{1,1}) et deuxième (u_{2,1}) trajectoires, il est possible de faire effectuer un mouvement de translation à la plateforme (4) dans la direction du premier axe (e_{x,w}) et dans la direction du deuxième axe (e_{y,w}).

6. Dispositif (1) selon l'une quelconque des revendications précédentes, dans lequel la plateforme (4) et les première (L_{1,1}) et deuxième (L_{2,1}) tiges forment un premier sous-châssis articulé (24) déplaçable par les premier (9) et deuxième (11) actionneurs afin de faire effectuer un mouvement de translation à la plateforme (Oₘ) dans la direction du premier axe (e_{x,w}) et dans la direction du deuxième axe (e_{y,w}),
dans lequel le premier sous-châssis articulé (24) forme de préférence une tringlerie trilatérale articulée.

7. Dispositif (1) selon l'une quelconque des revendications précédentes, dans lequel la plateforme (4) et la troisième tige (L_{3,1}), et au moins l'une parmi la première (L_{1,1}) et la deuxième (L_{2,1}) tige forment un deuxième sous-châssis articulé (25) déplaçable par les troisième (13), premier (9) et/ou deuxième (11) actionneurs afin de faire effectuer un mouvement de translation à la plateforme (4) dans la direction du premier axe (e_{x,w}) et dans la direction du troisième axe (e_{z,w}),
dans lequel le deuxième sous-châssis articulé (25) forme de préférence une tringlerie quadrilatérale articulée.

8. Dispositif (1) selon l'une quelconque des revendications précédentes, dans lequel les axes longitudinaux des quatrième (L4) et cinquième (L₅) tiges sont orientés et espacés, de sorte que, en ajustant la position des premières extrémités des quatrième (L₄) et cinquième (L₅) tiges le long de la quatrième (u_{4,1}) et de la cinquième (u_{5,1}) trajectoire, il est possible de faire tourner la plateforme (4) autour du troisième axe (e_{z,w}).

9. Dispositif (1) selon l'une quelconque des revendications précédentes, dans lequel la plateforme (4) et les quatrième (L₄) et cinquième (L₅) tiges forment un troisième sous-châssis articulé (26) déplaçable par le quatrième (15) et le cinquième (17) actionneur afin de faire tourner la plateforme (4) autour du troisième axe (e_{z,w}),
dans lequel le troisième sous-châssis articulé (26) forme de préférence une tringlerie quadrilatérale articulée.

10. Dispositif (1) selon la revendication 9, dans lequel la plateforme (4) a une forme de (T) avec une partie longitudinale (28) formant le centre de plateforme (Oₘ), et une partie transversale (27) s'étendant de manière transversale par rapport à l'extension de la partie longitudinale (28), dans lequel la partie transversale (27) forme deux extrémités opposées espacées du centre de plateforme (Oₘ) à la fois dans la direction du deuxième axe (e_{y,w}) et dans la direction du troisième axe (e_{z,w}), dans lequel la seconde extrémité de la quatrième tige (L4) et la seconde extrémité de la cinquième tige (L₅) sont raccordées respectivement en rotation à l'une des extrémités opposées de la partie transversale (27).

11. Dispositif (1) selon l'une quelconque des revendications précédentes, dans lequel, dans toute la plage de positionnement de la plateforme (4), les saillies orthogonales de l'axe longitudinal d'au moins l'une parmi la première (L_{1,1}) et la deuxième (L_{2,1}) tige et au moins l'une parmi la quatrième (L₄) et la cinquième (L₅) tige sur le plan défini par le premier axe (e_{x,w}) et le troisième axe (e_{z,w}) sont parallèles et espacées ou se coupent en un point espacé du centre de plateforme (Oₘ), de sorte que, en ajustant la position des premières extrémités d'au moins l'une parmi la première (L_{1,1}) et la deuxième (L_{2,1}) tige et d'au moins l'une parmi la quatrième (L₄) et la cinquième (L₅) tige le long des trajectoire (u_{1,1}), (_{U2,1}), (u_{4,1}), (u_{5,1}) respectives, il est possible de faire tourner la plateforme (4) autour du deuxième axe (e_{y,w}).

12. Dispositif (1) selon l'une quelconque des revendications précédentes, dans lequel au moins l'une parmi la première (L_{1,1}) et la deuxième (L_{2,1}) tige et au moins l'une parmi la quatrième (L₄) et la cinquième (L₅) tige forment, conjointement avec la plateforme (4), au moins un quatrième sous-châssis articulé (29) déplaçable par les actionneurs (9, 11, 15, 17) respectifs afin de faire tourner la plateforme (4) autour du deuxième axe (e_{y,w}), dans lequel le quatrième sous-châssis (29) forme une liaison quadrilatérale articulée.

13. Dispositif (1) selon l'une quelconque des revendications précédentes, dans lequel :
la première tige (L_{1,1}) et la deuxième tige (L_{2,1}) forment, au niveau de leurs extrémités, des joints à cardan rotatifs qui sont configurés pour transmettre des couples à la tige, tout en permettant la rotation autour de deux axes qui sont mutuellement orthogonaux et orthogonaux à un axe longitudinal de la tige respective.

14. Dispositif (1) selon l'une quelconque des revendications précédentes, dans lequel :
le premier bras (8) comprend en outre un premier bras de levier (30) raccordé de manière rotative à la première selle (P₁),
dans lequel la première extrémité de la première tige (L_{1,1}) est raccordée de manière rotative à une première extrémité du premier bras de levier (30), et une première extrémité d'une première tige supplémentaire (L_{1,2}) est raccordée de manière rotative à une seconde extrémité du premier bras de levier (30),
dans lequel la seconde extrémité de la première tige (L_{1,1}) est raccordée à la plateforme (4) dans un premier point de raccordement (32) et une seconde extrémité de la première tige supplémentaire (L_{1,2}) est raccordée à la plateforme (4) dans un deuxième point de raccordement (33),
de sorte que le premier bras (8) et la plateforme (4) forment une tringlerie quadrilatérale articulée,
le deuxième bras (10) comprend en outre un second bras de levier (31) qui est raccordé de manière rotative à la deuxième selle (P₂),
dans lequel la première extrémité de la deuxième tige (L_{2,1}) est raccordée de manière rotative à une première extrémité du second bras de levier (31), et une première extrémité d'une deuxième tige supplémentaire (L_{2,2}) est raccordée de manière rotative à une seconde extrémité du second bras de levier (31),
dans lequel la seconde extrémité de la deuxième tige (L_{2,1}) est raccordée à la plateforme (4) dans un troisième point de raccordement (34), et une seconde extrémité de la deuxième tige supplémentaire (L_{2,2}) est raccordée de manière rotative à la plateforme (4) dans un quatrième point de raccordement (35),
de sorte que le deuxième bras (10) et la plateforme forment une tringlerie quadrilatérale articulée,
dans lequel le troisième point de raccordement (34) est immédiatement adjacent au premier point de raccordement (32), et le quatrième point de raccordement (35) est immédiatement adjacent au deuxième point de raccordement (33), et les premier, deuxième, troisième et quatrième points de raccordement sont tous agencés sur un axe longitudinal de la plateforme (4), permettant une rotation des secondes extrémités des première, deuxième, troisième et quatrième tiges par rapport à la plateforme (4) autour de l'axe longitudinal de la plateforme (4),
dans lequel le premier bras de levier (30) et le deuxième bras de levier (31) sont couplés mutuellement par un joint à double cardan homocinétique d'une longueur variable (36) et configuré afin de maintenir les axes longitudinaux du premier bras de levier (30) et du second bras de levier (31) toujours parallèles.

15. Dispositif (1) selon l'une des revendications précédentes, comprenant un sixième bras (37) ayant une sixième selle (P₆) qui est raccordée de manière coulissante à la structure de support (2) le long d'une sixième trajectoire, et pouvant être positionnée le long de la sixième trajectoire par un sixième actionneur, ainsi qu'une sixième tige rigide ayant une première extrémité raccordée de manière rotative à la sixième selle (P₆) et une seconde extrémité raccordée de manière rotative à la plateforme (4),
dans lequel, dans toute la plage de positionnement de la plateforme (4), les axes longitudinaux des troisième (L₃) et sixième (L₆) tiges sont orientés et espacés, de sorte que, en ajustant la position des premières extrémités des troisième (L₃) et sixième (L₆) tiges le long des troisième (u_{3,1}) et sixième trajectoires, il est possible de faire tourner la plateforme (4) autour du premier axe (e_{x,W}).
